# EUROPEAN PATENT SPECIFICATION

(11) **EP 4 340 914 B1**
(45) Date of publication and mention of the grant of the patent: **18.11.2026**
(21) Application number: 22728923.8
(22) Date of filing: 16.05.2022
(51) Int. Cl.: A61M 15/00

(54) **INHALER WITH AN ADHERENCE / COMPLIANCE MONITOR**
INHALATOR MIT EINEM ADHÄRENZ-/COMPLIANCE-MONITOR
INHALATEUR DOTÉ D'UN MONITEUR DE CONFORMITÉ/D'ADHÉRENCE

(30) Priority: 17.05.2021 EP 21174149
(43) Date of publication of application: 27.03.2024
(73) Proprietor: Vectura Delivery Devices Limited, Chippenham, Wiltshire SN14 6FH (GB)
(72) Inventor: SMITH, Philip, Cambridge Cambridgeshire CB4 0GZ (GB); SWANBURY, Philip, Cambridge Cambridgeshire CB4 0GZ (GB); MELINIOTIS, Andreas, Cambridge Cambridgeshire CB05 0GZ (GB); MORRIS, Danny, Stafford Staffordshire ST18 0AR (GB); DUNKLEY, Jack, Stafford Staffordshire ST18 0AR (GB); HEYNES, Mark, Stafford Staffordshire ST18 0AR (GB)
(74) Representative: Clarke, Christopher John
(86) International application number: PCT/EP2022/063134
(87) International publication number: WO 2022/243220

(56) References cited:
- WO-A1-2015/109259
- WO-A1-2015/174856
- WO-A1-2015/178907
- WO-A1-2016/049066
- WO-A1-2016/111633
- WO-A2-2014/204511
- US-A1- 2010 192 948

## Description

### Technical Field of the Invention

The present invention relates to an inhaler containing an active substance for inhalation and a monitor having one or more sensors for monitoring a patient's adherence and / or compliance. In particular, the invention relates to an inhaler with a monitor with a mounting mechanism that is configurable so that the monitor can be either detachably or permanently attached to the inhaler.

### Background to the Invention

Inhalers, such as dry powder inhalers (DPIs), provide an attractive method for administering medicaments, for example to treat local diseases of the airway or to deliver drugs to the bloodstream via the lungs. The medicament is commonly provided as individual doses, such as a strip having a plurality of blisters. The dose is typically dispensed by the user opening a cap or cover to access a mouthpiece, then operating an actuator, such as a button or lever to release the powder, and finally inhaling through the mouthpiece. In some inhalers (known as "open-inhale-close" inhalers) the cover itself is the actuator, so that there is no separate actuating lever or button. The inhalers usually have a dose counter which displays the number of doses that have been used, or that remain to be used.

The efficacy of treatment is dependent on the patient using the inhaler correctly and as prescribed. Consequently, there is increasing interest in monitoring patient adherence (i.e. whether the patient takes the prescribed number of doses per day, e.g. once or twice daily) and compliance (i.e. whether the patient uses their inhaler correctly, e.g. if they inhale sufficiently strongly to entrain the powder and disperse it into particles that reach the lung).

DPIs typically contain a month's supply of medication. Since adherence / compliance monitors usually contain expensive sensors, electronics etc., they are often provided as separate add-on modules which detachably couple to the inhaler. Thus, when the medication in the inhaler has been used up, the monitor can be detached and then re-attached to a new inhaler. For example, WO 2014/204511 discloses adherence monitors for the Diskus^{®} dry powder inhaler, which may be configured as a casing that fits over the top and bottom portions of the inhaler. The monitor is therefore easy to install and remove. WO 2015/178907 discloses a monitor in the form of an external casing into which the inhaler is inserted. The monitor has friction screws which are tightened in order to secure the monitor to the inhaler. The monitor is therefore removable by loosening the screws. US 2010/192948 discloses a monitor that is releasably attachable to an inhaler.

Alternatively, the monitor may be permanently attached to the inhaler, so that it cannot be inadvertently removed by the user. This ensures that the monitor records adherence / compliance information throughout the lifetime of the inhaler. For example, EP 3552647 discloses a built-in monitor for a Diskus^{®}-type inhaler. The monitor is fixedly mounted to the casing of the inhaler under a cover unit by means of screws. EP 3551263 discloses an integrated monitor for a Diskus^{®}-type inhaler which is located inside the housing of the inhaler. These monitors are not removable.

Thus both removable and permanently attached monitors are required, for different users and / or different products. WO 2016/111633, WO 2016/049066, WO 2015/174856, and WO 2015/109259 disclose monitors that may be removably or permanently attached to inhalers. It would be advantageous to provide a monitor for an inhaler which can be easily configured to be either permanently attached or removable without having to change the design of the monitor.

### Brief description of the invention

The invention is defined by the appended claims.

The present invention addresses this problem and, in a first aspect, provides an inhaler with a monitor having one or more sensors, wherein the monitor is attachable to the inhaler by a mounting mechanism comprising at least one pair of complementary mounting members, wherein one mounting member of the or each pair of mounting members is located on the inhaler and the other mounting member of the or each pair of mounting members is located on the monitor; wherein the mounting mechanism, in particular one or more of the mounting members, is configurable by moving a component of that mounting member or by removing a detachable part of that mounting member, so that the monitor can be detachably mounted on the inhaler in a first configuration of the configurable mounting member(s) and can be permanently attached to (i.e. mounted onto) the inhaler in a second configuration of the configurable mounting member(s).

Each pair of mounting members may comprise a male mounting member and a female mounting member.

The monitor may comprise a male mounting member and the inhaler may comprise a female mounting member, or the inhaler may comprise a male mounting member and the monitor may comprise a female mounting member.

In contrast to the known monitors which are retro-fitted (either detachably or permanently) onto pre-existing inhalers, the inhaler and monitor of the present invention are designed from the start to be used with each other. This allows the mounting mechanism to be configurable so that the monitor can be attached to the inhaler in either a detachable or permanent manner. The single base design provides both the removable and the fixed options, and only a simple additional step is necessary during assembly to select the desired configuration.

In one embodiment, the monitor has two male mounting members and the inhaler has two female mounting members. One or both of the male mounting members may be configurable so that the monitor can be either detachably or permanently attached to (i.e. mounted onto) the inhaler.

The configurable male mounting member may be a clip comprising a primary clip member and a secondary clip member. The corresponding female mounting member may be a slot.

In the first configuration, when the monitor is detachably mounted on the inhaler, the primary clip member engages with the slot and the secondary clip member does not engage with the primary clip member and / or the slot. In the second configuration, when the monitor is permanently mounted on the inhaler, the primary clip member engages with the slot and the secondary clip member engages with the primary clip member and / or the slot.

The secondary clip member may be configurable, e.g. movable from a first position in which it does not engage with the primary clip member and / or the slot to a second position in which it engages with the primary clip member and / or the slot. The secondary clip member may be movable via an orifice in the housing of the inhaler or in the casing of the monitor, for example during assembly of the inhaler. Alternatively, the secondary clip member may be configurable by removing a breakable / detachable part.

The primary clip member may have a body and an enlarged head which acts as a pawl, and the corresponding slot may have a lip and a void, which forms a detent mechanism for holding the head of the primary clip member inside the slot so that the monitor can be mounted on the inhaler and can be detached from the inhaler with a small force (such as less than 30 N).

The secondary clip member may have a head and the primary clip member may have a step. When the monitor is permanently mounted on the inhaler, the head of the secondary clip member may engage with the step the primary clip member as a ratchet and pawl. The head of the secondary clip member may act as the pawl which engages with the step on the primary clip member that acts as the ratchet.

When the monitor is permanently mounted on the inhaler, the secondary clip member may prevent the primary clip member from moving so that the detent mechanism cannot be released. In particular, the head of the secondary clip member may be located inside the slot and may prevent the head of the primary clip member from overcoming the detent mechanism. Thus the monitor can only be detached from the inhaler with a large force (such as greater than 150N).

The secondary clip member may have a flexible body. The secondary clip member may be a separate component. The secondary clip member may have a removable part.

In a second aspect, the invention provides a monitor for an inhaler, the monitor having one or more sensors, wherein the monitor has at least one configurable mounting member for attaching the monitor to the inhaler by engaging with a corresponding slot in the inhaler, wherein the configurable mounting member is configurable by moving a component of the mounting member or by removing a detachable part of the mounting member so that the monitor can be detachably mounted on the inhaler in a first configuration of the configurable mounting member(s) and can be permanently mounted on the inhaler in a second configuration of the configurable mounting member(s), wherein the at least one configurable mounting member comprises a primary clip member and a secondary clip member.

In a third aspect, the invention provides an inhaler for use with a monitor having one or more sensors, wherein the inhaler has at least one configurable mounting member for attaching the monitor to the inhaler by engaging with a corresponding slot in the monitor, wherein the configurable mounting member is configurable by moving a component of the mounting member or by removing a detachable part of the mounting member so that the monitor can be detachably mounted on the inhaler in a first configuration of the configurable mounting member(s) and can be permanently mounted on the inhaler in a second configuration of the configurable mounting member(s), wherein the at least one configurable mounting member comprises a primary clip member and a secondary clip member.

### Brief Description of the Figures

The invention will now be further described with reference to the Figures, wherein:
Figure 1A shows an inhaler and a monitor according to the invention, with the cover in the closed position, so that the mouthpiece is covered.
Figure 1B shows the inhaler of Figure 1A with the cover in the open position.
Figure 1C shows the inhaler of Figure 1A without the monitor and with the cover closed.
Figure 1D shows the inhaler of Figure 1A without the monitor and with the cover open.
Figures 2A and 2B show the monitor.
Figure 3 shows a first clip.
Figures 4A, 4B and 4D show a second clip; Figure 4C shows the orifice for configuring the second clip.
Figures 5A and 5B show a second embodiment of the second clip.
Figures 6A and 6B show a third embodiment of the second clip.
Figures 7A and 7B show a fourth embodiment of the second clip.
Figures 8A and 8B show a fifth embodiment of the second clip.

### Detailed Description of the Invention

In the context of inhalers, the term "adherence" is normally used to refer to whether the patient takes the prescribed number of doses per day, e.g. once or twice daily. The term "compliance" is normally used to refer to whether the patient uses their inhaler correctly, e.g. if they inhale sufficiently strongly to entrain the powder and disperse it into particles that reach the lung. Consequently, a monitor may be designed to measure adherence and / or compliance, according to the type of sensors that it uses, and how they are configured. In the present application, the term "monitor" therefore refers to a module having one or more sensors that is designed to measure and capture information relating to adherence and / or compliance. However, the monitor does not perform any of the functions associated with dosing the medication, such as a piercing or opening blisters / capsules, de-agglomerating the powder or providing a breath-actuation mechanism. The inhaler therefore operates to dispense the active substance whether the monitor is present or not.

The term "mounting mechanism" refers to a mechanism for mechanically connecting the inhaler and monitor using at least one pair of mounting members with complementary shape such as to engage with each other, wherein one mounting member of each pair is located on the inhaler, and the other is located on the monitor. One of the members (often referred to as "male") is inserted, clicked or hooked into, and/or caught by, the other member (often referred to as "female"). A male member has a particular shape (such as as a peg or other protrusion) adapted for mechanical engagement with a complementary female member (such as a slot or other cavity). Thus the mounting mechanism locates the monitor in a predefined position with respect to the inhaler and provides a retaining force to hold it in position. For example, the mounting mechanism may comprise a clip (male feature) in the form of protrusion with an enlarged head and a corresponding slot (female feature) with a lip. Typically, the male member is somewhat flexible, and, in the detachable configuration, release is easy and achieved e.g. by slightly pressing or pulling the monitor, such as in the case of a snap-fit connection. In the permanent configuration, the movement of the male member is restricted so that it cannot flex, thereby preventing detachment of the monitor.

The term "detachably attached" means that the monitor is intended to be removable by a user, for example using their fingers. This term does not refer to monitors that are intended not to be removable by a user, and which are only removable by applying a very large force, such as with a tool. The terms "removable" and "detachable" are used interchangeably.

The term "permanently attached" means that the monitor is not intended to be removable by a user, for example using their fingers. However, this term does not require that it is completely impossible to remove the monitor (so for example, a permanently attached monitor might be removable by using tools).

The term "configurable" refers to a mounting member, or component thereof, which is designed to have two different positions or states. The state can be selected or changed, in particular during assembly of the inhaler and the monitor. The selection or change can be effected for example by moving (such as bending, flexing, translating) the component; or by removing (such as breaking off) a detachable part of the component.

The inhaler of the invention preferably has a strip of blisters containing powdered medicament, and a mechanism for advancing the blister strip and for opening the blisters which is operated by an actuator. The opening mechanism is suitably a piercer which is mounted on the underside of the mouthpiece. The actuator drives the indexing mechanism to move a blister into alignment with the piercer and then moves the mouthpiece relative to the housing so that the piercer pierces the aligned blister. The actuator may be a lever which causes indexing of the blister strip and piercing of the blisters. Preferably however, the actuator is formed as part of, or is connected to, the cover, so that rotation of the cover causes indexing of the blister strip and piercing of the blisters. The inhaler may be configured to index and pierce one blister on each actuation. Alternatively, it may index and pierce two (or more) blisters on each actuation. For example, it may deliver two (or more) different formulations or medicaments simultaneously.

However, the invention is not limited to this type of inhaler, and for example, could equally be an inhaler which has a passive mouthpiece cover, and a separate actuating lever, as described for example in WO13/175176, or with an inhaler which has a blister disk instead of a blister strip, or a reservoir DPI or a capsule DPI. Moreover, the invention can equally apply to other types of inhaler, such as pressurized metered dose inhalers (pMDI) or soft mist inhalers.

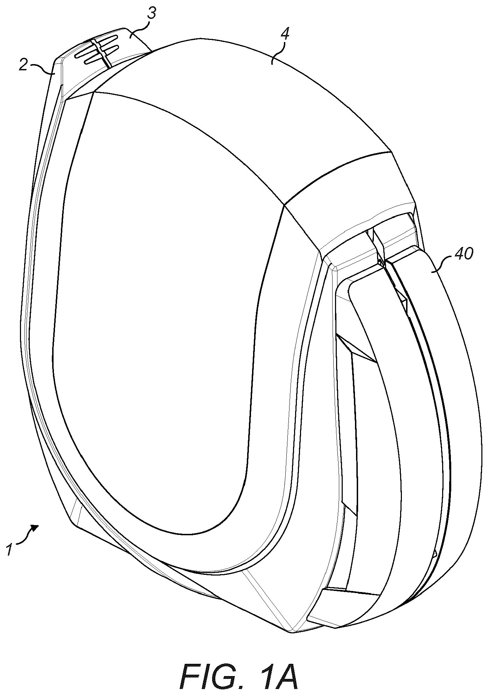

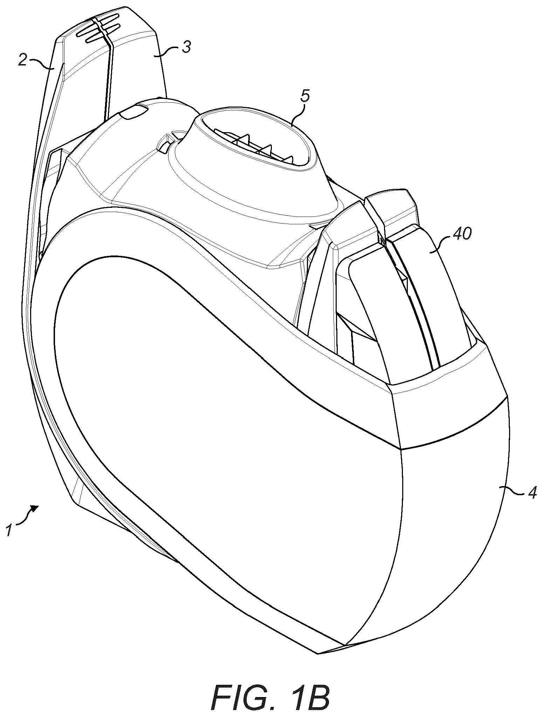

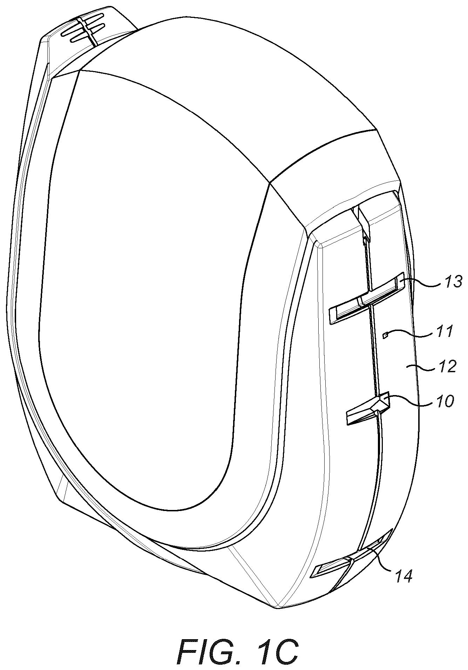

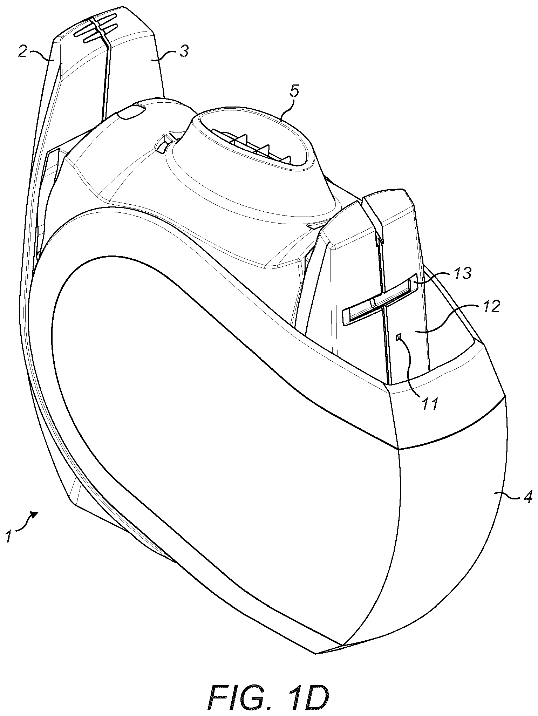

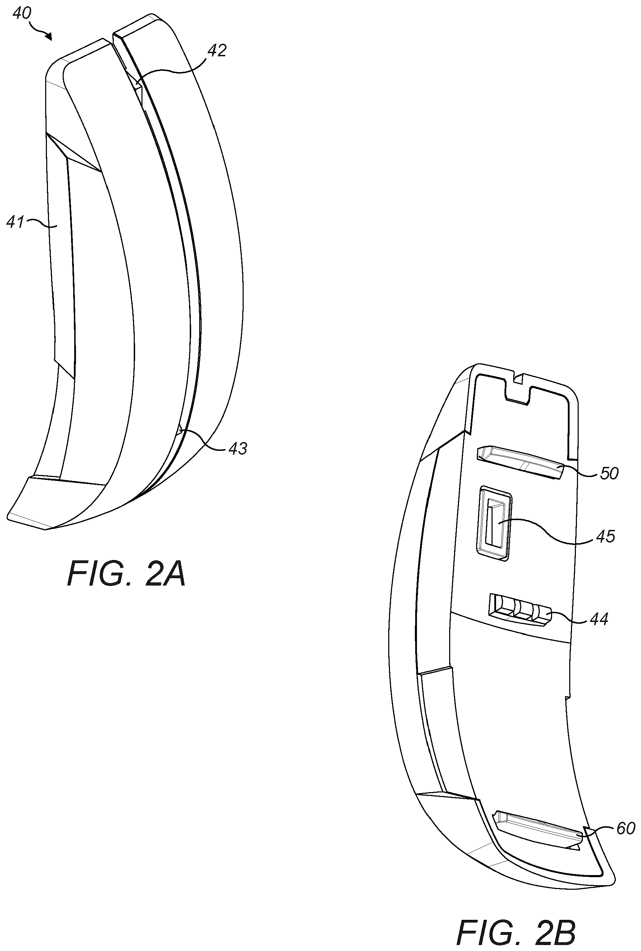

An inhaler and monitor of the invention are shown in Figures 1 and 2 . Figure 1A shows the inhaler with a monitor attached, and with the mouthpiece cover in the closed position. Figure 1B shows the inhaler with the mouthpiece cover in the open position so that the mouthpiece is visible. Figures 1C and 1D show the inhaler with the monitor removed and with the mouthpiece cover in the closed and open positions respectively.

The inhaler shown in Figure 1 is an "open-inhale-close" dry powder inhaler of the type described in WO13/175177, which has a gear mechanism that selectively couples the mouthpiece cover to a blister strip indexing mechanism and also to a piercer. Moving the cover from the closed position to an intermediate position causes the indexing mechanism to advance the blister strip. Then, once an unused blister is in position beneath the piercer, the indexing mechanism is disengaged. Moving the mouthpiece cover from the intermediate position to the fully open position causes the piercer to pierce the aligned blister. The user then inhales through the mouthpiece, which aerosolizes the powder in the pierced blister.

The inhaler **1** shown in Figure 1 is constructed from two shell parts **2, 3** which are joined together to form a housing that contains a blister strip. A monitor **40** is attached to one side of the inhaler. A mouthpiece cover **4** is mounted onto the housing. The cover **4** can be rotated through approximately 100° from the closed position (Figure 1A) in which it covers and protects a mouthpiece, to a fully open position (Figure 1B), in which the mouthpiece 5 is exposed so that the user can inhale a dose of medicament. When the cover is in the open position for inhalation, the monitor is situated under the cover, so that it is protected from accidental damage.

The inner face of the monitor matches the shape of the housing of the inhaler on which it is mounted. The outer face corresponds to the curve defined by the rotation of cover. In other words, it is shaped as an arc of a circle centred on the axis of rotation of the cover, with a radius which is slightly less than the radius of the internal surface of the cover. Thus, when the cover is opened there is a small clearance gap (about 0.5 - 1mm) between the internal surface of the cover and outer face of the monitor. This maximises the volume of the monitor within the constraint that it can fit under the cover.

Figures 1C and 1D show the inhaler without the monitor. The inhaler has two slots **13, 14** for mounting the monitor (the second slot **14** is not visible in Figure 1D because it is hidden by the cover **4).** There are two orifices **10, 11** in the wall **12** of the housing where the monitor was attached whose purposes are described below.

Figures 2A and 2B show the monitor **40,** which has a casing **41** containing a circuit board and one or more sensors. The outside face of the monitor shown in Figure 2A has a sensor or switch **42** which is used to determine whether the mouthpiece cover has been opened. It also has an orifice **43** whose purpose is explained below.

The inner face of the monitor (i.e. the side which abuts the inhaler when the monitor is attached) is shown in Figure 2B. The monitor has three optical sensors **44** which protrude into the orifice **10** on the inhaler when the monitor is attached to the inhaler. The optical sensors read a code on the blister strip (for example, a printed bar code) so that the monitor can determine the number of doses that have been dispensed or that remain to be dispensed. The monitor also has a pressure sensor **45,** which is is located in a recess on the inner face. The pressure sensor abuts the orifice **11,** which leads, via a channel in the housing, to the mouthpiece. The monitor can measure the pressure in the mouthpiece and thereby detect the user's inhalation. The monitor **40** also has two clips **50, 60** which fit into the corresponding slots **13, 14** in the housing of the inhaler, and thereby hold the monitor in place when attached to the inhaler. The first (upper) clip **50** is stiff and the second (lower) clip **60** is flexible (although equally the upper clip could be flexible and the lower clip could be stiff).

The monitor may be configured so that it is detachable and hence may be transferred to a new inhaler once the medication in the original inhaler has been used up. In this detachable configuration, the monitor can be removed with a force of less than 30N, which is low enough for elderly or infirm users to be able to detach it. Alternatively, the monitor may be configured so that it is permanently attached to the inhaler. In this fixed configuration, the monitor is capable of resisting attempted removal by a user, and requires a force of e.g. greater than 150N to remove it. These two configurations are achieved from a single design by designing the second clip **60** so that it can be configured during the factory assembly process in either the permanently mounted state or the detachably mounted state.

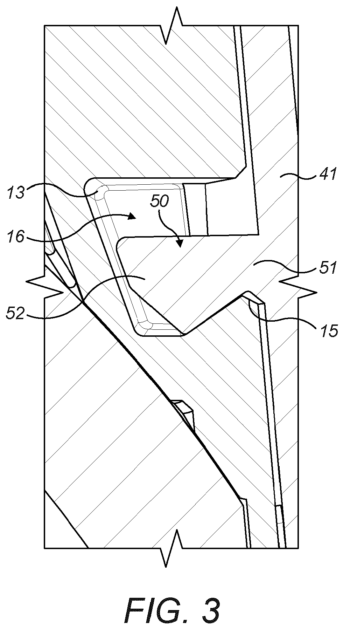

Figure 3 is a cross-sectional view through the inhaler and the monitor in the region of the first slot **13** and the first clip **50.** It shows the first clip **50** in place in the first slot **13** when the monitor is attached to the inhaler. The first clip **50** protrudes from the casing **41** of the monitor, and has a neck **51** and an enlarged head **52.** The first slot **13** has a lip **15** on its lower side and a void **16.** To attach the monitor to the inhaler, the head **52** is first inserted into the slot **13** over the lip **15.** Then it is pulled downwards in order to line up the second clip with the second slot for insertion (described below) so that the head **52** is located behind the lip **15.** The first clip **50** is rigid, so that the lip **15** retains the head **52** in place. If the second clip is released from the second slot, the head **52** is then free to rise up over the lip **15** and the first clip **50** can be removed from the first slot **13.**

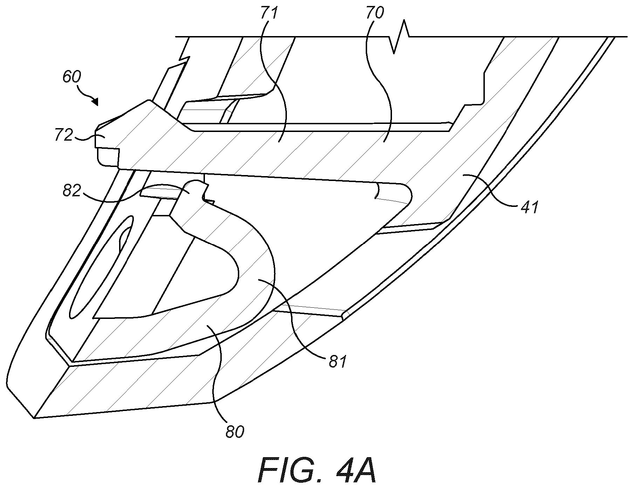

Figure 4A is a cross-sectional view through the lower part of the monitor showing the second clip **60** in detail. The second clip **60** has a primary clip member **70** with a generally planar body **71** and an enlarged head **72.** The body **71** is located inside the casing **41** of the monitor, and the head **72** protrudes outside the casing. The second clip **60** also has a secondary clip member **80** with an arcuate body **81** and a head **82,** also located inside the casing. The secondary clip member **80** is flexible due to the arcuate shape of the body so that the head **82** can be moved into a position in which it protrudes outside of the casing, in a manner that is explained below.

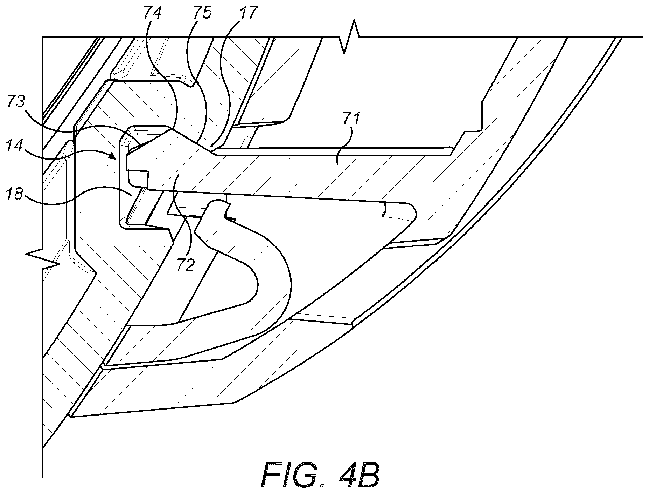

Figure 4B shows the second clip engaged with the second slot in the removable configuration. In this configuration, the secondary clip member is passive and remains inside the casing of the monitor. The second slot **14** has a lip **17** on its upper side and a void **18.** When the monitor is attached to the inhaler, the head **72** of the primary clip member **70** is inserted into the slot **14.** The head **72** has an inverted "V" shaped profile, so that the front face **73** of the head slopes upwards to a ridge **74,** and the rear face **75** of the head slopes downwards from the ridge **74.** The body **71** is resilient, so that as the head **72** is inserted into the slot **14,** the lip **17** pushes against the sloping front face **73,** and the head **72** is deflected downwards into the void **18,** until the lip **17** passes over the ridge **74.** The resilience of the body **71** causes the head to deflect back upwards as the rear face **75** moves over the lip. Thus the lip **17** together with the resilient body **71** provides a detent mechanism for holding the head **72,** which acts as a pawl, inside the slot **14.** This, together with the first clip **50** in the first slot **13,** holds the monitor in place on the inhaler during use.

In this configuration, the insertion process is reversible: as the head **72** is removed from the slot **14,** the lip **17** pushes against the sloping rear face **75,** the head **72** is deflected downwards into the void **18,** until the ridge **74** rides over the lip **17.** The resilience of the body **71** causes the head to deflect back upwards as the front face **73** moves over the lip. Thus sufficient force must be applied to the monitor for the body **71** to be deflected so that the head **72** passes over the lip, i.e. in order to overcome the hold placed on the head by the detent mechanism. Once the second clip has been released from the second slot, the monitor can be moved upwards a short distance so that the first clip can be removed from the first slot. In this manner, the monitor can be detached from the inhaler with a relatively small force (such as less than 30N), e.g. once all of the doses have been inhaled.

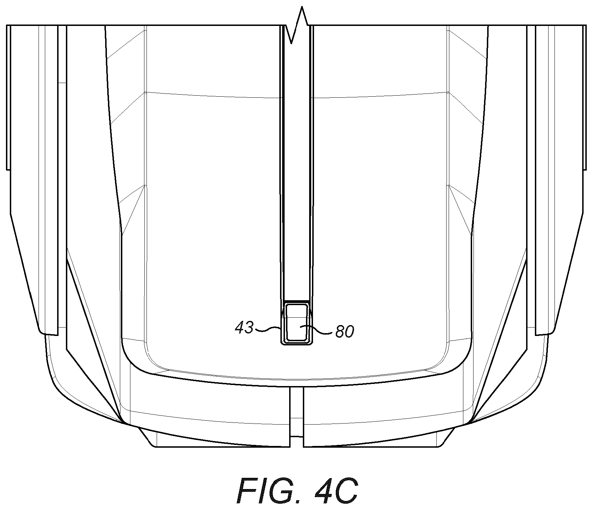

Figure 4C shows the lower part of the outer side of the monitor **40** attached to the inhaler. The secondary clip member **80** is visible through the orifice **43.** In order to change from the detachably mounted configuration to the permanently attached configuration, a rod is inserted though the orifice **43** (e.g. during assembly of the inhaler) so that it comes into contact with and pushes against the body **81** of the secondary clip member. The secondary clip member flexes so that the head **82** is moved out of the casing **41** of the monitor and into the second slot **14.**

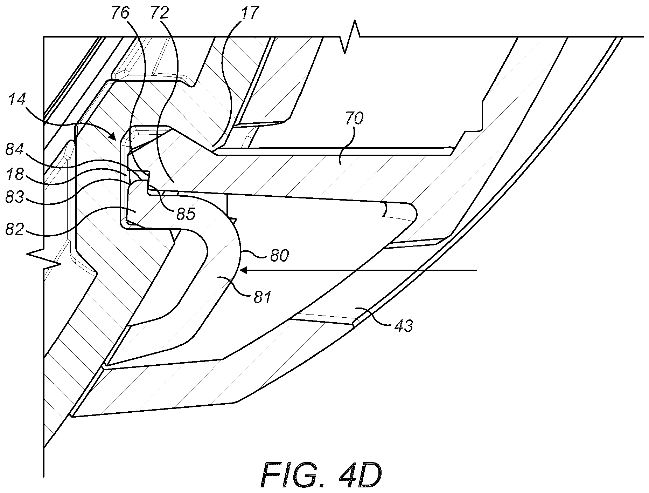

Figure 4D shows the second clip engaged with the second slot in the fixed (i.e. permanently attached) configuration. The head **72** of the primary clip member **70** is inserted behind the lip **17** of the slot **14** as described above for the detachable configuration. Then, the secondary clip member **80** is pushed via the orifice **43** (indicated by the arrow) so that the head **82** is inserted into the void **18** beneath the head **72** of the primary clip member **70.** The lower side of the head **72** of the primary clip member **70** has a step **76.** The front face **83** of the head **82** of the secondary clip member **80** slopes upwards to a plateau **84,** and the rear face **85** forms a step downwards from the plateau **84.** The arcuate body **81** is flexible, so that as the head **82** is inserted into the void **18,** the sloping front face **83** pushes against the lower side of the head **72** of the primary clip member **70.** This deflects the head **82** downwards, until it reaches the step **76.** Once the head **82** has passed over the step **76,** the resilience of the arcuate body **81** causes the head **82** to deflect upwards into the void **18** beyond the step **76.** The head **82** is then located behind the step **76.** Since the rear face **85** of the head is vertical (unlike the rear face **75** of the head **72** of the primary clip member **70),** the head **82** is held behind the step **76.** Thus the step **76** on the head **72** of the primary clip member **70** and the head **82** of the secondary clip member **80** provide a ratchet and pawl. The body **81** of the secondary clip member **80** is located beneath the head **72** of the primary clip member **70** and prevents the head **72** from being deflected downwards if the user attempts to remove the monitor from the inhaler. In other words, the secondary clip member **80** is locked in place by the head **82** and the step **76;** this in turns holds the head **72** of the primary clip member **70** in place behind the lip **17.** A much larger force (such as greater than 150N) is thus required to remove the monitor, so that it is essentially permanently attached.

In an alternative arrangement, the step **76** could be provided on the lower surface of the slot **14,** and the head **82** of the secondary clip member **80** could protrude downwardly so that when the secondary clip member **80** is inserted into the slot **14,** the head **82** would again be held behind the step **76.**

In summary, when the secondary clip member is not inserted into the slot (Figure 4B), the second clip is able to flex, which permits removal of the monitor with a force of less than 30N. To change to the fixed configuration, the secondary clip member is inserted into the slot, for example during the assembly process after the monitor has been attached. This locks the second clip in place so that it is no longer able to flex, thereby preventing removal of the monitor. Thus the second clip can be easily configured so that the force required to remove the monitor is greater than 150N.

The user should not be able to inadvertently or misguidedly change from the detachable configuration to the fixed configuration whilst using the monitor. Thus the secondary clip member should be sufficiently flexible that it can be pushed into the fixed configuration during assembly, but sufficiently stiff that a user is not able to push it into the fixed configuration by for example, poking a pin into the aperture. Suitably, the force required to push the secondary clip member into the fixed configuration is greater than 300N.

The monitor can be attached to the inhaler by inserting the first clip into the first slot, and then the second clip into the second slot as described above. Alternatively, the second clip can first be inserted into the second slot, and then subsequently the first clip can be inserted into the first slot. If the monitor is to be permanently attached to the inhaler, the second clip is placed into the fixed configuration by pushing the secondary clip member into position in the second slot via the orifice.

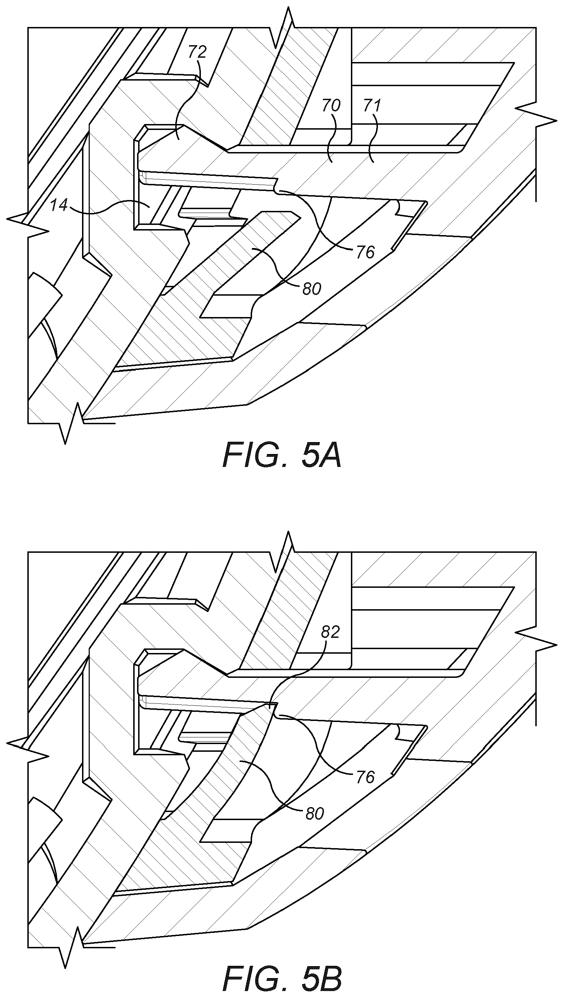

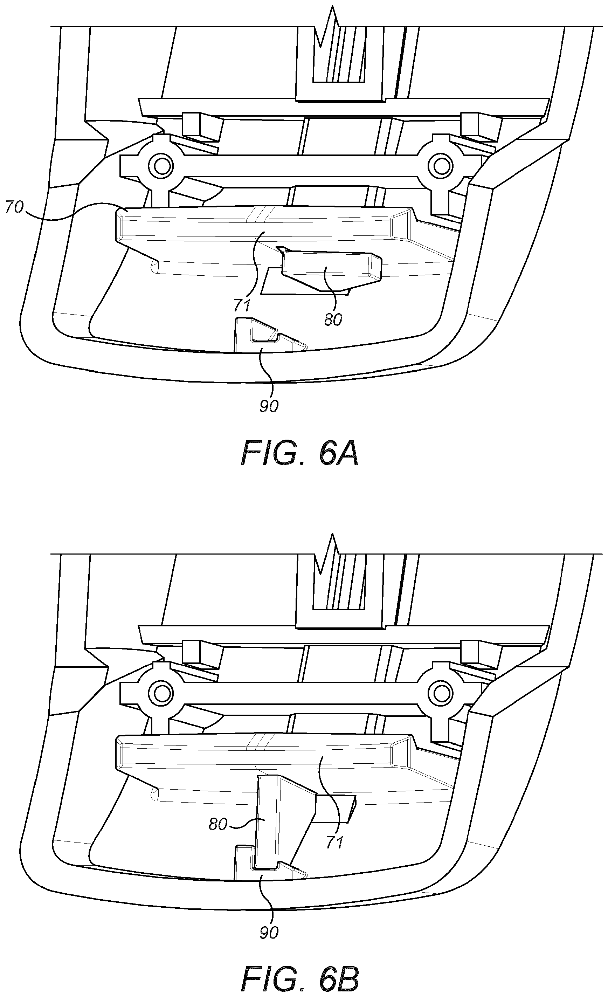

The secondary clip member could interact with the primary clip member outside the slot rather than inside it, as shown in Figures 5 and 6. In the embodiment of Figure 5, the primary clip member **70** has a step **76** located on its body **71** rather than on the head **72** , and the secondary clip member **80** is movable from a position in which it does not interact with the primary clip member **70** (the detachable configuration, shown in Figure 5A) into a position in which its head **82** engages with the step **76** and holds the primary clip member **70** in place (the permanently attached configuration, as shown in Figure 5B) without entering the slot **14.** Figure 6 shows an alternative embodiment in which the secondary clip member **80** is a living hinge which is rotated from a position in which it does not interact with the primary clip member **70** (the detachable configuration, shown in Figure 6A) into a position in which it holds the body **71** of the primary clip member **70** in place (the permanently attached configuration, shown in Figure 6B). Instead of a step on the primary clip member, the casing of the monitor has a detent feature **90** which holds the secondary clip member **80** in place.

In an alternative embodiment, the secondary clip member is a separate component rather than an integral part of the second clip. Thus, when assembling the monitor for the detachable configuration, the secondary clip member is not present at all. On the other hand, when assembling the monitor for the fixed configuration, the secondary clip member is inserted into the slot. The secondary clip member may be included in the clip assembly before mounting the monitor onto the inhaler and then inserted into the slot, for example by pushing a rod through the aperture to move the secondary clip member into position in the slot. Alternatively, the secondary clip member could itself be inserted through the aperture and into the slot. Since the secondary clip member is not present at all in the detachable configuration, there is no possibility of the user inadvertently changing from the detachable configuration to the fixed configuration whilst using the monitor, even if the user poked a pin into the aperture.

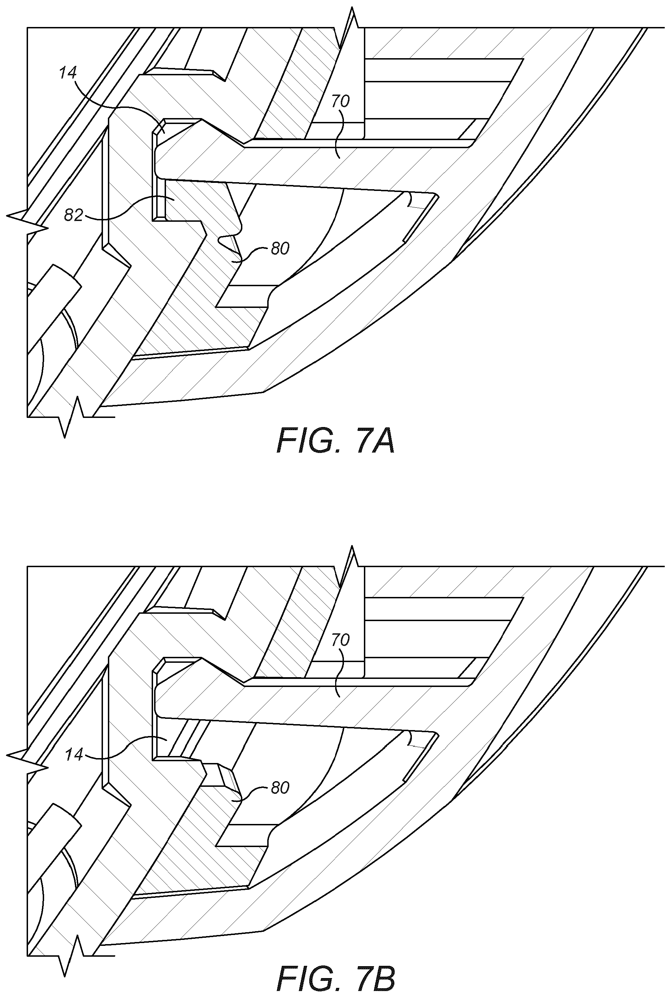

Instead of the secondary clip member being movable, it could be rigid (so that it is not movable), but have a part which can be broken off or otherwise removed before or during assembly in order to change from the permanently attached configuration to the removable configuration. For example, in the embodiment shown in Figure 7, the secondary clip member **80** has a detachable head **82.** When the is present (Figure 7A), it locks the primary clip member **70** in place in the slot **14** so that the monitor is permanently mounted on the inhaler. When the head has been removed (Figure 7B), there is nothing to lock the primary clip member **70** in the slot **14,** so that the monitor is detachably mounted on the inhaler.

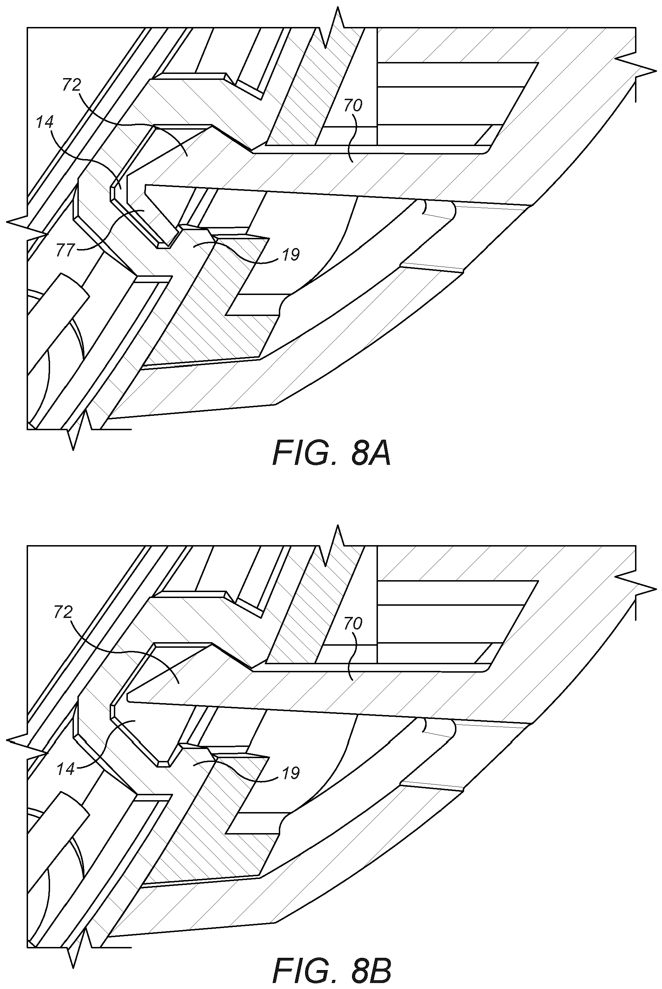

In yet another embodiment, shown in Figure 8, there is no secondary clip member; instead, the head **72** of the primary clip member **70** has a removable tooth **77** which, when present, engages with a lip **19** on the lower side of the slot **14** to lock the primary clip member **70** in place (Figure 8A), so that the monitor is permanently attached to the inhaler. Similar to the previous embodiment, removing the tooth before assembly prevents the primary clip member **70** from being locked in the slot **14** (Figure 8B), so that the monitor is detachably mounted.

In the embodiment shown in Figures 1 to 4, there are two pairs of mounting members. However, the inhaler and monitor could have only one pair of mounting members (e.g. one male mounting member, such as a clip, and one female mounting member, such as a slot); alternatively, the inhaler and monitor could have three or more pairs of mounting members.

In the embodiment shown in Figures 1 to 4, the monitor has the clips (male mounting members) and the inhaler has the slots (female mounting members). This has the advantage that there are no clips protruding from the inhaler, so that the inhaler is more comfortable to hold if it is used without the monitor. Moreover, it is easy to access the secondary clip member through the aperture in the casing of the monitor in order to place the second clip into the fixed configuration. Nonetheless, the monitor could instead have the slots and the inhaler could have the clips.

It is only necessary for one of the mounting members to be configurable; nonetheless several or all of the mounting members may be configurable. For example, in the embodiment described above, only the second (lower) clip is configurable. However, the first (upper) clip could alternatively or additionally be configurable. Having more than one configurable mounting member has the advantage that an even greater force would be required to remove the monitor in the fixed configuration; however, increasing the number of configurable mounting members may increase the cost and the complexity of assembly.

In the embodiment shown in Figures 1 to 4, the first (non-configurable) clip and the first slot help to retain the monitor in place by means of the enlarged head of the clip and the lip of the slot. This is advantageous, since having two pairs of mounting members which retain the monitor in place provides for secure attachment (in either the detachable or the fixed configuration). However, this is not essential and in another embodiment, the first clip and slot could simply help to position the monitor in place on the inhaler without providing any retention. For example, the clip could be a simple protrusion (without an enlarged head) and the slot could have no lip. In this embodiment, only the second (configurable) clip would hold the monitor on the inhaler.

In the embodiment shown in Figures 1 to 4, the configurable mounting member is a male mounting member. However, a female mounting member could alternatively or additionally be configurable, for example by having a flexible or movable secondary slot member which could be moved into the void in order to lock the primary clip member in place.

The invention provides a monitor which can be either detachably or permanently attached to an inhaler using a simple assembly process. Creating the permanent configuration needs only a simple additional step (e.g. pushing a component with a rod) and does not require, for example, a welding or gluing step.

The monitor may have a controller and memory (e.g. a suitable microprocessor) which are configured to process and/or store information read from the sensors relating to patient's usage of the inhaler. The monitor may also include communication means to transmit adherence / compliance information (e.g. via bluetooth) to an external device, such as a computer or smartphone. The information may then be displayed to the patient and / or a medical professional, by means of suitable software, for example a smartphone app. The information may additionally or alternatively be stored on the monitor for subsequent interrogation, or wirelessly transmitted to, for example, an online health platform.

The medicament is suitable for administration by inhalation, for example for the treatment of a respiratory disease. It may include one of more of the following classes of pharmaceutically active material: anticholinergics, adenosine A2A receptor agonists, β2-agonists, calcium blockers, IL-13 inhibitors, phosphodiesterase-4-inhibitors, kinase inhibitors, steroids, CXCR2, proteins, peptides, immunoglobulins such as Anti-IG-E, nucleic acids in particular DNA and RNA, monoclonal antibodies, small molecule inhibitors and leukotriene B4 antagonists. The medicament may include excipients, such as fine excipients and / or carrier particles (for example lactose), and / or additives (such as magnesium stearate, phospholipid or leucine).

Suitable β2-agonists include albuterol (salbutamol), e.g. albuterol sulfate; carmoterol, e.g. carmoterol hydrochloride; fenoterol; formoterol; milveterol, e.g. milveterol hydrochloride; metaproterenol, e.g. metaproterenol sulfate; olodaterol; procaterol; salmeterol, e.g. salmeterol xinafoate; terbutaline, e.g. terbutaline sulphate; vilanterol, e.g. vilanterol trifenatate or indacaterol, e.g. indacaterol maleate. Suitable steroids include budesonide; beclamethasone, e.g. beclomethasone dipropionate; ciclesonide; fluticasone, e.g. fluticasone furoate; mometasone, e.g. mometasone furoate. Suitable anticholinergics include: aclidinium, e.g. aclidinium bromide; glycopyrronium, e.g. glycopyrronium bromide; ipratropium, e.g. ipratropium bromide; oxitropium, e.g. oxitropium bromide; tiotropium, e.g. tiotropium bromide; umeclidinium, e.g. umeclidinium bromide; Darotropium bromide; or tarafenacin.

The active material may include double or triple combinations such as salmeterol xinafoate and fluticasone propionate; budesonide and formoterol fumarate dehydrate; glycopyrrolate and indacaterol maleate; glycopyrrolate, indacaterol maleate and mometasone furoate; fluticasone furoate and vilanterol; vilanterol and umeclidinium bromide; fluticasone furoate, vilanterol and umeclidinium bromide.

## Claims

1. An inhaler (1) having a housing (2,3) and a monitor (40) having a casing and one or more sensors (44, 45), wherein the monitor is attachable to the inhaler by a mounting mechanism comprising at least one pair of complementary mounting members, wherein the inhaler comprises one mounting member (13,14) of the or each pair of mounting members and the monitor comprises the other mounting member (50,60) of the or each pair, **characterized in that** at least one of the mounting members is configurable by moving a component of that mounting member or by removing a detachable part of that mounting member, so that the monitor can be detachably mounted on the inhaler in a first configuration of the configurable mounting member(s) and can be permanently mounted on the inhaler in a second configuration of the configurable mounting member(s).

2. An inhaler with a monitor according to claim 1 wherein the or each pair of mounting members comprise a male mounting member and a female mounting member.

3. An inhaler with a monitor according to claim 1 or claim 2, wherein the monitor has two male mounting members (50,60) and the inhaler has two female mounting members (13,14), and wherein one of the male mounting members (60) is configurable.

4. An inhaler with a monitor according to claim 3, wherein the configurable male mounting member (60) comprises a primary clip member (70) and a secondary clip member (80), and the corresponding female mounting member is a slot (14).

5. An inhaler with a monitor according to claim 4, wherein:
• in the first configuration the primary clip member (70) engages with the slot (14) and the secondary clip member (80) does not engage with the primary clip member (70) and / or the slot (14), so that the monitor is detachably mounted on the inhaler; and wherein
• in the second configuration the primary clip member (70) engages with the slot (14) and the secondary clip member (80) engages with the primary clip member (70) and / or the slot (14), so that the monitor is permanently mounted on the inhaler.

6. An inhaler with a monitor according to claim 4 or claim 5, wherein the secondary clip member is movable from a first position in which it does not engage with the primary clip member and / or the slot to a second position in which it engages with the primary clip member and / or the slot.

7. An inhaler with a monitor according to claim 6 wherein the secondary clip member is movable via an orifice (43) in the housing of the inhaler or in the casing of the monitor.

8. An inhaler with a monitor according to any of claims 4 to 7, wherein the primary clip member (70) has a body (71) and a head (72), and the corresponding slot (14) has a lip (17) and a void (18), which together form a detent mechanism for holding the head of the primary clip member inside the slot.

9. An inhaler with a monitor according to claim 8, wherein, when the monitor is permanently mounted on the inhaler, the secondary clip member prevents the primary clip member from moving so that the detent mechanism cannot be released.

10. An inhaler with a monitor according to claim 9, wherein the secondary clip member (80) has a head (82), and wherein when the monitor is permanently mounted on the inhaler, the head (82) of the secondary clip member (80) engages with a step (76) on the primary clip member (70) as a ratchet and pawl.

11. An inhaler with a monitor according to claim 10, wherein when the monitor is permanently mounted on the inhaler, the head (82) of the secondary clip member (80) is located inside the slot (14).

12. An inhaler with a monitor according to any of claims 4 to 11, wherein the secondary clip member (80) has a flexible body (81).

13. An inhaler with a monitor according to any of claims 4 to 11, wherein the secondary clip member (80) is a separate component or has a removable part.

14. A monitor (40) for an inhaler (1), the monitor having one or more sensors (44,45) and at least one configurable mounting member (60) for attaching the monitor to the inhaler by engaging with a corresponding slot in the inhaler, wherein the configurable mounting member is configurable by moving a component of the mounting member or by removing a detachable part of the mounting member so that the monitor can be detachably mounted on the inhaler in a first configuration of the configurable mounting member(s) and can be permanently mounted on the inhaler in a second configuration of the configurable mounting member(s), and wherein the configurable mounting member(s) comprises a primary clip member (70) and a secondary clip member (80).

15. An inhaler (1) for use with a monitor (40) having one or more sensors (44,45), wherein the inhaler has at least one configurable mounting member for attaching the monitor to the inhaler by engaging with a corresponding slot in the monitor, wherein the configurable mounting member is configurable by moving a component of the mounting member or by removing a detachable part of the mounting member so that the monitor can be detachably mounted on the inhaler in a first configuration of the configurable mounting member(s) and can be permanently mounted on the inhaler in a second configuration of the configurable mounting member(s), and wherein the configurable mounting member(s) comprises a primary clip member and a secondary clip member.

## Patentansprüche

1. Inhalator (1) mit einem Gehäuse (2,3) und einem Monitor (40), der ein Gehäuse und einen oder mehreren Sensoren (44, 45) aufweist, wobei der Monitor durch einen Montagemechanismus, der wenigstens ein Paar von komplementären Montageelementen umfasst, an dem Inhalator befestigbar ist, wobei der Inhalator ein Montageelement (13,14) des oder jedes Paars von Montageelementen umfasst und der Monitor das andere Montageelement (50,60) des oder jedes Paars umfasst, **dadurch gekennzeichnet, dass** wenigstens eines der Montageelemente durch Bewegen einer Komponente dieses Montageelements oder durch Entfernen eines ablösbaren Teils dieses Montageelements konfigurierbar ist, so dass der Monitor in einer ersten Konfiguration des/der konfigurierbaren Montageelemente ablösbar an dem Inhalator montiert werden kann und in einer zweiten Konfiguration des/der konfigurierbaren Montageelemente dauerhaft an dem Inhalator montiert werden kann.

2. Inhalator mit einem Monitor nach Anspruch 1, wobei das oder jedes Paar von Montageelementen ein männliches Montageelement und ein weibliches Montageelement umfasst.

3. Inhalator mit einem Monitor nach Anspruch 1 oder Anspruch 2, wobei der Monitor zwei männliche Montageelemente (50,60) aufweist und der Inhalator zwei weibliche Montageelemente (13,14) aufweist und wobei eines der männlichen Montageelemente (60) konfigurierbar ist.

4. Inhalator mit einem Monitor nach Anspruch 3, wobei das konfigurierbare männliche Montageelement (60) ein primäres Clip-Element (70) und ein sekundäres Clip-Element (80) umfasst und das entsprechende weibliche Montageelement ein Schlitz (14) ist.

5. Inhalator mit einem Monitor nach Anspruch 4, wobei:
• in der ersten Konfiguration das primäre Clip-Element (70) in den Schlitz (14) eingreift und das sekundäre Clip-Element (80) nicht mit dem primären Clip-Element (70) und/oder dem Schlitz (14) Eingriff bildet, so dass der Monitor ablösbar an dem Inhalator montiert wird; und wobei
• in der zweiten Konfiguration das primäre Clip-Element (70) in den Schlitz (14) eingreift und das sekundäre Clip-Element (80) mit dem primären Clip-Element (70) und/oder dem Schlitz (14) Eingriff bildet, so dass der Monitor dauerhaft an dem Inhalator montiert wird.

6. Inhalator mit einem Monitor nach Anspruch 4 oder Anspruch 5, wobei das sekundäre Clip-Element von einer ersten Position, in der es nicht mit dem primären Clip-Element und/oder dem Schlitz Eingriff bildet, in eine zweite Position, in der es mit dem primären Clip-Element und/oder dem Schlitz Eingriff bildet, bewegbar ist.

7. Inhalator mit einem Monitor nach Anspruch 6, wobei das sekundäre Clip-Element über eine Öffnung (43) in dem Gehäuse des Inhalators oder in dem Gehäuse des Monitors bewegbar ist.

8. Inhalator mit einem Monitor nach einem der Ansprüche 4 bis 7, wobei das primäre Clip-Element (70) einen Körper (71) und einen Kopf (72) aufweist und der entsprechende Schlitz (14) eine Lippe (17) und einen Hohlraum (18) aufweist, die zusammen einen Arretiermechanismus zum Halten des Kopfs des primären Clip-Elements in dem Schlitz bilden.

9. Inhalator mit einem Monitor nach Anspruch 8, wobei, wenn der Monitor dauerhaft an dem Inhalator montiert ist, das sekundäre Clip-Element verhindert, dass sich das primäre Clip-Element bewegt, so dass der Arretiermechanismus nicht gelöst werden kann.

10. Inhalator mit einem Monitor nach Anspruch 9, wobei das sekundäre Clip-Element (80) einen Kopf (82) aufweist und wobei, wenn der Monitor dauerhaft an dem Inhalator montiert ist, der Kopf (82) des sekundären Clip-Elements (80) als Ratsche und Klinke mit einer Stufe (76) an dem primären Clip-Element (70) in Eingriff steht.

11. Inhalator mit einem Monitor nach Anspruch 10, wobei, wenn der Monitor dauerhaft an dem Inhalator montiert ist, der Kopf (82) des sekundären Clip-Elements (80) in dem Schlitz (14) angeordnet ist.

12. Inhalator mit einem Monitor nach einem der Ansprüche 4 bis 11, wobei das sekundäre Clip-Element (80) einen flexiblen Körper (81) aufweist.

13. Inhalator mit einem Monitor nach einem der Ansprüche 4 bis 11, wobei das sekundäre Clip-Element (80) eine getrennte Komponente ist oder ein entfernbares Teil aufweist.

14. Monitor (40) für einen Inhalator (1), wobei der Monitor einen oder mehrere Sensoren (44,45) und wenigstens ein konfigurierbares Montageelement (60) zum Befestigen des Monitors an dem Inhalator durch Eingreifen in einen entsprechenden Schlitz in dem Inhalator aufweist, wobei das konfigurierbare Montageelement durch Bewegen einer Komponente des Montageelements oder durch Entfernen eines ablösbaren Teils des Montageelements konfigurierbar ist, so dass der Monitor in einer ersten Konfiguration des/der konfigurierbaren Montageelemente ablösbar an dem Inhalator montiert werden kann und in einer zweiten Konfiguration des/der konfigurierbaren Montageelemente dauerhaft an dem Inhalator montiert werden kann, und wobei das/die konfigurierbaren Montageelemente ein primäres Clip-Element (70) und ein sekundäres Clip-Element (80) umfassen.

15. Inhalator (1) zur Verwendung mit einem Monitor (40), der einen oder mehrere Sensoren (44,45) aufweist, wobei der Inhalator wenigstens ein konfigurierbares Montageelement zum Befestigen des Monitors an dem Inhalator durch Eingreifen in einen entsprechenden Schlitz in dem Monitor aufweist, wobei das konfigurierbare Montageelement durch Bewegen einer Komponente des Montageelements oder durch Entfernen eines ablösbaren Teils des Montageelements konfigurierbar ist, so dass der Monitor in einer ersten Konfiguration des/der konfigurierbaren Montageelemente ablösbar an dem Inhalator montiert werden kann und in einer zweiten Konfiguration des/der konfigurierbaren Montageelemente dauerhaft an dem Inhalator montiert werden kann, und wobei das/die konfigurierbaren Montageelemente ein primäres Clip-Element und ein sekundäres Clip-Element umfassen.

## Revendications

1. Inhalateur (1) ayant un carter (2, 3) et un dispositif de surveillance (40) ayant un boîtier et un ou plusieurs capteurs (44, 45), le dispositif de surveillance pouvant être fixé à l'inhalateur par un mécanisme de montage comprenant au moins une paire d'éléments de montage complémentaires, l'inhalateur comprenant un élément de montage (13, 14) de la ou de chaque paire d'éléments de montage et le dispositif de surveillance comprenant l'autre élément de montage (50, 60) de la ou de chaque paire, **caractérisé en ce qu'**au moins un des éléments de montage est configurable par déplacement d'un composant de cet élément de montage ou par retrait d'une partie amovible de cet élément de montage, de sorte que le dispositif de surveillance puisse être monté de manière amovible sur l'inhalateur dans une première configuration de l'élément ou des éléments de montage configurables et puisse être monté de manière permanente sur l'inhalateur dans une seconde configuration de l'élément ou des éléments de montage configurables.

2. Inhalateur doté d'un dispositif de surveillance selon la revendication 1, la ou chaque paire d'éléments de montage comprenant un élément de montage mâle et un élément de montage femelle.

3. Inhalateur doté d'un dispositif de surveillance selon la revendication 1 ou la revendication 2, le dispositif de surveillance ayant deux éléments de montage mâles (50, 60) et l'inhalateur ayant deux éléments de montage femelles (13, 14), et l'un des éléments de montage mâles (60) étant configurable.

4. Inhalateur doté d'un dispositif de surveillance selon la revendication 3, l'élément de montage mâle configurable (60) comprenant un élément d'attache primaire (70) et un élément d'attache secondaire (80), et l'élément de montage femelle correspondant étant une fente (14).

5. Inhalateur doté d'un dispositif de surveillance selon la revendication 4 :
• dans la première configuration, l'élément d'attache primaire (70) venant en prise avec la fente (14) et l'élément d'attache secondaire (80) ne venant pas en prise avec l'élément d'attache primaire (70) et/ou la fente (14), de sorte que le dispositif de surveillance est monté de manière amovible sur l'inhalateur ; et
• dans la seconde configuration, l'élément d'attache primaire (70) venant en prise avec la fente (14) et l'élément d'attache secondaire (80) venant en prise avec l'élément d'attache primaire (70) et/ou la fente (14), de sorte que le dispositif de surveillance est monté de manière permanente sur l'inhalateur.

6. Inhalateur doté d'un dispositif de surveillance selon la revendication 4 ou la revendication 5, l'élément d'attache secondaire étant mobile d'une première position, dans laquelle il ne vient pas en prise avec l'élément d'attache primaire et/ou la fente, à une seconde position, dans laquelle il vient en prise avec l'élément d'attache primaire et/ou la fente.

7. Inhalateur doté d'un dispositif de surveillance selon la revendication 6, l'élément d'attache secondaire étant mobile par l'intermédiaire d'un orifice (43) ménagé dans le carter de l'inhalateur ou dans le boîtier du dispositif de surveillance.

8. Inhalateur doté d'un dispositif de surveillance selon l'une quelconque des revendications 4 à 7, l'élément d'attache primaire (70) ayant un corps (71) et une tête (72), et la fente correspondante (14) ayant une lèvre (17) et un creux (18), qui forment ensemble un mécanisme d'encliquetage pour maintenir la tête de l'élément d'attache primaire à l'intérieur de la fente.

9. Inhalateur doté d'un dispositif de surveillance selon la revendication 8, lorsque le dispositif de surveillance est monté de manière permanente sur l'inhalateur, l'élément d'attache secondaire empêchant l'élément d'attache primaire de se déplacer, de sorte que le mécanisme d'encliquetage ne peut pas être libéré.

10. Inhalateur doté d'un dispositif de surveillance selon la revendication 9, l'élément d'attache secondaire (80) ayant une tête (82), et, lorsque le dispositif de surveillance est monté de manière permanente sur l'inhalateur, la tête (82) de l'élément d'attache secondaire (80) venant en prise avec un gradin (76) de l'élément d'attache primaire (70) en formant un mécanisme à rochet et cliquet.

11. Inhalateur doté d'un dispositif de surveillance selon la revendication 10, lorsque le dispositif de surveillance est monté de manière permanente sur l'inhalateur, la tête (82) de l'élément d'attache secondaire (80) étant située à l'intérieur de la fente (14).

12. Inhalateur doté d'un dispositif de surveillance selon l'une quelconque des revendications 4 à 11, l'élément d'attache secondaire (80) ayant un corps flexible (81).

13. Inhalateur doté d'un dispositif de surveillance selon l'une quelconque des revendications 4 à 11, l'élément d'attache secondaire (80) étant un composant distinct ou ayant une partie pouvant être retirée.

14. Dispositif de surveillance (40) pour un inhalateur (1), le dispositif de surveillance ayant un ou plusieurs capteurs (44, 45) et au moins un élément de montage configurable (60) pour fixer le dispositif de surveillance à l'inhalateur en venant en prise avec une fente correspondante de l'inhalateur, l'élément de montage configurable étant configurable par déplacement d'un composant de l'élément de montage ou par retrait d'une partie amovible de l'élément de montage, de sorte que le dispositif de surveillance puisse être monté de manière amovible sur l'inhalateur dans une première configuration de l'élément ou des éléments de montage configurables et puisse être monté de manière permanente sur l'inhalateur dans une seconde configuration de l'élément ou des éléments de montage configurables, et l'élément ou les éléments de montage configurables comprenant un élément d'attache primaire (70) et un élément d'attache secondaire (80).

15. Inhalateur (1) destiné à être utilisé avec un dispositif de surveillance (40) ayant un ou plusieurs capteurs (44, 45), l'inhalateur ayant au moins un élément de montage configurable pour fixer le dispositif de surveillance à l'inhalateur en venant en prise avec une fente correspondante du dispositif de surveillance, l'élément de montage configurable étant configurable par déplacement d'un composant de l'élément de montage ou par retrait d'une partie amovible de l'élément de montage, de sorte que le dispositif de surveillance puisse être monté de manière amovible sur l'inhalateur dans une première configuration de l'élément ou des éléments de montage configurables et puisse être monté de manière permanente sur l'inhalateur dans une seconde configuration de l'élément ou des éléments de montage configurables, et l'élément ou les éléments de montage configurables comprenant un élément d'attache primaire et un élément d'attache secondaire.
